# EUROPÄISCHE PATENTSCHRIFT

(11) **EP 1 262 171 B1**
(45) Veröffentlichungstag und Bekanntmachung des Hinweises auf die Patenterteilung: **10.05.2006**
(21) Anmeldenummer: 02011419.5
(22) Anmeldetag: 24.05.2002
(51) Int. Cl.: A61K 8/04, A61K 8/34, A61K 8/41, C07C 211/63, C11D 1/62, C11D 3/20

(54) **konfektionierte quaternäre Ammoniumzusammensetzungen**
formulated quaternary ammonium compositions
compositions d'ammonium quaternaire

(30) Priorität: 29.05.2001 DE 10126253
(43) Veröffentlichungstag der Anmeldung: 04.12.2002
(73) Patentinhaber: Clariant Produkte (Deutschland) GmbH, 65929 Frankfurt am Main (DE)
(72) Erfinder: Milbradt, Robert, Dr., 65189 Wiesbaden (DE); Klein, Sonja, 65795 Hattersheim (DE); Scherl, Franz Xaver, Dr., 84508 Burgkirchen (DE); Gatter, Erich, Dr., 84556 Kastl (DE); Oberhauser, Adelgunde, 84524 Neuötting (DE)

(56) Entgegenhaltungen:
- WO-A-00/28950
- WO-A-91/12880
- US-A- 5 102 655
- US-A- 5 888 488
- US-A- 5 929 263
- DATABASE WPI Section Ch, Week 199444 Derwent Publications Ltd., London, GB; Class D21, AN 1994-354596 XP002213719 -& JP 06 279233 A (HOYU KK), 4. Oktober 1994 (1994-10-04)

## Beschreibung

Die Erfindung betrifft Zusammensetzungen, enthaltend quaternäre Ammoniumverbindungen, die einen niedrigen Stockpunkt, gute Löslichkeit bzw. Dispergierbarkeit in wässrigen Medien und einen niedrigen Flammpunkt besitzen und sich somit hervorragend zur Konfektionierung von quaternären Ammoniumverbindungen eignen.

Kosmetische Mittel, wie z.B. Haarbehandlungsmittel, enthalten häufig in Wasser schwerlösliche quaternäre Ammoniumverbindungen, die eine langkettige Alkyl- oder Alkenylgruppe aufweisen. Solche Mittel sind üblicherweise als wässrige Dispersionen, Emulsionen, Mikroemulsionen, Gele oder auch in Aerosolform konfektioniert und werden z.B. als Shampoos, Haarkuren, Haarspülungen etc. eingesetzt.

Für den Hersteller solcher Mittel ist es von großem Vorteil, die quaternären Ammoniumverbindungen als Compounds oder Formulierungen in Form von Flakes, Pellets oder Pasten bereitzustellen, die bei hohem kationischen Wirkstoffanteil einen niedrigen Stockpunkt und gute Löslichkeit bzw. Dispergierbarkeit in wässrigen Medien besitzen.
Gemäß dem Stand der Technik können die obigen Anforderungen durch die Zugabe von kurzkettigen Alkoholen, insbesondere von Isopropanol in Mengen von 15 bis 20 Gew.-%, erreicht werden. Aufgrund ihrer niedrigen Siede- und Flammpunkte ist der Einsatz solcher kurzkettiger Alkohole jedoch problematisch.
Wie in WO 00/28950 beschrieben, können die kurzkettigen Alkohole durch lineare Fettalkohole (z.B. Cetyl-, Lauryl-, Behenyl- oder Stearylalkohol) ersetzt werden. Um den Stockpunkt der Mischungen auf Temperaturen unterhalb 100°C herabzusetzen werden zusätzlich Glykole, wie z.B. Propylenglykol oder 1,3-Butandiol, zugesetzt.
In der WO 00/28950 wird weiterhin hervorgehoben, dass es sich bei den Fettalkoholen vorteilhafterweise um homogene Fettalkohole handelt, die weniger als ca. 10 Gew.-% eines anderen Fettalkohols enthalten.

Überraschenderweise wurde nun gefunden, dass Zusammensetzungen, enthaltend quaternäre Ammoniumverbindungen, verzweigte langkettige Alkohole, bevorzugt Mischungen von verzweigten und unverzweigten langkettigen Alkoholen, und niedermolekulare mehrwertige Alkohole, einen niedrigen Stockpunkt, gute Löslichkeit bzw. Dispergierbarkeit in wässrigen Medien und einen niedrigen Flammpunkt besitzen. Derartige Zusammensetzungen eignen sich damit hervorragend zur Konfektionierung von quaternären Ammoniumverbindungen.

Gegenstand der Erfindung sind Zusammensetzungen, enthaltend, bezogen auf die fertigen Zusammenstungen
a) 30 bis 90 Gew- % mindestens eine quaternäre Ammoniumverbindung gemäß Formel (1) 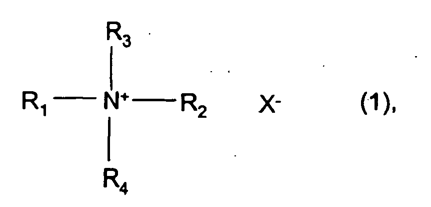 wobei
   R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ steht, wobei R₅ für eine Alkyl- oder Alkenylgruppe mit 12 bis 36 Kohlenstoffatomen und n für eine Zahl von 1 bis 8 stehen,
   und
   R₂, R₃ und R₄ unabhängig voneinander gleich oder verschieden sein können und für eine -CH₃, CH₃CH₂-, CH₃CH₂CH₂-, CH₃CH₂CH₂CH₂-, CH₂CH₂OH oder
   CH₂CH(OH)CH₂OH-Gruppe stehen
   und
   X⁻ für ein Anion steht,
   und
b) eine Mischung aus mindestens einem verzweigten Alkohol b1) mit 8 bis 36 Kohlenstoffatomen und mindestens einem unverzweigten Alkohol b2) mit 8 bis 36 Kohlenstoffatomen, wobei das Gewidits verhältnis b1) zu b2) 90:10 bis 30:70 beträgt, und
c) mindestens einen mehrwertigen Alkohol mit 2 bis 6 Kohlenstoffatomen dadurch gekennzeichnet, dass die in Form von Pellets oder Flakes vorliegen.

Die verzweigten Alkohole b1) und unverzweigten Alkohole b2) können gesättigt oder ungesättigt sein.

Der Anteil an quaternären Ammoniumverbindungen a), bezogen auf die fertigen Zusammensetzungen, beträgt 30 bis 90 Gew.-%, bevorzugt 50 bis 90 Gew.-%, besonders bevorzugt 60 bis 90 Gew.-%, ganz besonders bevorzugt 60 bis 80 Gew.-%.
Überraschenderweise zeigte sich, dass die Zusammensetzungen vorteilhafterweise hohe Gewichtsanteile an quaternären Ammoniumverbindungen a) bei gleichzeitig niedrigen Schmelz- bzw. Stockpunkten enthalten können.

Als quaternäre Ammoniumverbindungen a) bevorzugt sind (C₁₂-C₃₆)-Alkyltrimethylammoniumverbindungen, besonders bevorzugt (C₁₄-C₃₀)-Alkyltrimethylammoniumverbindungen, insbesondere bevorzugt (C₁₆-C₂₄)-Alkyltrimethylammoniumverbindungen.
Insbesondere bevorzugt sind Alkyltrimethylammoniumverbindungen bei denen der Alkylrest einen Behenyl-, Erucyl-, Cetyl- oder Stearylrest darstellt.
Beim Anion X⁻ in Formel (1) kann es sich um ein beliebiges ladungsausgleichendes Anion handeln; bevorzugt sind Chlorid, lodid, Bromid, Methosulfat, Hydrogensulfat, Lactat und/oder Citrat, besonders bevorzugt Chlorid und Methosulfat.
Ganz besonders geeignet als quaternäre Ammoniumverbindung a) ist Behenyltrimethylammoniumchlorid.

Der Anteil der Alkoholkomponente b) beträgt, bezogen auf die fertigen Zusammensetzungen, bevorzugt 10 bis 60 Gew.-%, besonders bevorzugt 10 bis 50 Gew.-%, insbesondere bevorzugt 20 bis 40 Gew.-%.

Enthalten die Zusammensetzungen verzweigte Alkohole b1) und unverzweigte Alkohole b2), dann beträgt das Gewichtsverhältnis von verzweigten Alkoholen b1) zu unverzweigten Alkoholen b2) 90 : 10 bis 30 : 70, bevorzugt 70 : 30 bis 30 : 70, insbesondere bevorzugt 60 : 40 bis 40 : 60. Bevorzugt besitzen die verzweigten Alkohole b1) und die unverzweigten Alkohole b2) 10 bis 24, besonders bevorzugt 12 bis 18, insbesondere bevorzugt 14 bis 16 Kohlenstoffatome. Die verzweigten Alkohole b1) sind bevorzugt einfach verzweigt.

Als verzweigte Alkohole b1) besonders geeignet sind Guerbetalkohole, 2-Methyl-1-decanol, 2-Ethyl-1-nonanol, 2-Propyl-1-octanol, 2-Butyloctanol, 2-Butyl-decanol, 2-Hexyloctanol, 2-Hexyldecanol, 2-Octyldecanol, 2-Hexyldodecanol, 2-Octyldodecanol, 2-Decyltetradecanol, 2-Butyl-1-heptanol, 2-Dodecylhexadecanol, Tetradecyloctadecanol, 2-Tetradecyleicosanol, 2-Hexadecyloctadecanol, 2-Hexadecyleicosanol, 2-Methyltridecanol, 2-Ethyldodecanol, 2-Propylundecanol, 2-Pentylnonanol, 2-Heptylheptanol, 2-Methyltetradecanol, 2-Ethyltridecanol, 2-Propyldodecanol, 2-Butylundecanol, 2-Pentyldecanol, 2-Hexylnonanol, 2-Heptyloctanol, Isotridecylalkohol und Mischungen derselben.

Besonders bevorzugt sind Zusammensetzungen, die als verzweigte Alkohole b1) Alkoholgemische entsprechend den kommerziell erhältlichen ® Isalchem-Typen (Fa. Sasol) enthalten.

Bevorzugt als verzweigte Alkohole b1) für den gemeinsamen Einsatz mit unverzweigten Alkoholen b2) sind 2-Methyltridecanol, 2-Ethyldodecanol, 2-Propylundecanol, 2-Butyldecanol, 2-Pentylnonanol, 2-Hexyloctanol, 2-Heptylheptanol, 2-Methyltetradecanol, 2-Ethyltridecanol, 2-Propyldodecanol, 2-Butylundecanol, 2-Pentyldecanol, 2-Hexylnonanol und 2-Heptyloctanol.

Bevorzugt als unverzweigte Alkohole b2) sind Fettalkohole, besonders bevorzugt Lauryl-, Myristyl-, Cetyl-, Stearyl, Eicosanyl- und Behenylalkohol. Ebenfalls bevorzugt sind Nonanol, Undecanol, Tridecanol, Pentadecanol und Heptadecanol.

In einer besonders bevorzugten Ausführungsform enthalten die Zusammensetzungen verzweigte Alkohole b1) und unverzweigte Alkohole b2) mit jeweils 14 bis 16 Kohlenstoffatomen, wobei das Gewichtsverhältnis, bezogen auf die fertigen Zusammensetzungen, von verzweigten Alkoholen b1) zu unverzweigten Alkoholen b2) 70 : 30 bis 30 : 70, besonders bevorzugt 65 : 35 bis 55 : 45, insbesondere bevorzugt 60 : 40 beträgt und das Gewichtsverhältnis von Alkoholen mit 14 Kohlenstoffatomen zu Alkoholen mit 15 Kohlenstoffatomen im Bereich von 55 : 45 bis 65 : 35 liegt.

Besonders bevorzugt sind Zusammensetzungen, die als Mischungen von verzweigten Alkoholen b1) und unverzweigten Alkoholen b2) Alkoholgemische entsprechend den ® LIAL-Typen (z.B. ® LIAL 145 und 123, Fa. Sasol) enthalten. Die Alkohole aus den ® LIAL-Typen werden typischerweise nach dem Oxo-Prozess hergestellt.

Der Anteil an mehrwertigen Alkoholen c) beträgt, bezogen auf die fertigen Zusammensetzungen, bevorzugt 0.5 bis 20 Gew.-%, besonders bevorzugt 0.5 bis 10 Gew.-%.
Unter mehrwertigen Alkoholen c) sind Alkohole mit mindestens zwei OH-Gruppen zu verstehen.
Bevorzugt sind Glykole, besonders bevorzugt Ethylenglykol und Propylenglykol, Butandiole, besonders bevorzugt 1,3-Butandiol, Glycerin, Thioglycerin, Sorbitol, Xylitol und Mannitol.
Besonders bevorzugt sind Propylenglykol, Glycerin und 1,3-Butandiol.

Optional können die erfindungsgemäßen Zusammensetzungen zur Verbesserung der anwendungstechnischen Eigenschaften unverzweigte oder verzweigte Monoalkohole mit 1 bis 4 Kohlenstoffatomen enthalten. Bevorzugt als Monoalkohole sind Ethanol, Propanol, Isopropanol, Butanol, Isobutanol und t-Butanol, besonders bevorzugt Isopropanol. Bevorzugt enthalten die Zusammensetzungen, bezogen auf die fertigen Zusammensetzungen, weniger als 5 Gew.-%, besonders bevorzugt weniger als 1 Gew.-% an derartigen Monoalkoholen. In einer ebenfalls bevorzugten Ausführungsform sind die Zusammensetzungen frei von derartigen Monoalkoholen.

Die erfindungsgemäßen Zusammensetzungen besitzen bevorzugt einen Stockpunkt unterhalb 100 °C, besonders bevorzugt unterhalb 95°C, insbesondere bevorzugt unterhalb 90°C, ganz besonders bevorzugt 85 °C.

Der Flammpunkt der erfindungsgemäßen Zusammensetzungen liegt bevorzugt oberhalb 80°C, besonders bevorzugt oberhalb 100°C.

Bei den erfindungsgemäßen Zusammensetzungen handelt es sich um Pellets und Flakes.

In einer bevorzugten Ausführungsform werden die erfindungsgemäßen Zusammensetzungen dadurch hergestellt, dass eine Mischung hergestellt wird, enthaltend
i) mindestens eine quaternäre Ammoniumverbindung a), gegebenenfalls enthaltend einen verzweigten oder unverzweigten Monoalkohol mit 1 bis 4 Kohlenstoffatomen,
ii) eine Mischung aus mindestens einem verzweigten Alkohol b1) mit 8 bis 36 Kohlenstoffatomen und mindestens einem unverzweigten Alkohol b2) mit 8 bis 36 Kohlenstoffatomen,
iii) mindestens einen mehrwertigen Alkohol c) und
iv) gegebenenfalls mindestens einen unverzweigten oder verzweigten Monoalkohol mit 1 bis 4 Kohlenstoffatomen.

In einer bevorzugten Ausführungsform werden die Komponenten i) bis iv) vermischt und anschließend, gegebenenfalls unter Rühren, erwärmt. Dabei wird die Temperatur so gewählt, dass eine Schmelze vorliegt. Bevorzugt sind Temperaturen von 70 bis 120 °C, besonders bevorzugt 80 bis 110°C. In einer anderen bevorzugten Ausführungsform wird die Komponente i) als Schmelze vorgelegt.

Die quaternären Ammoniumverbindungen a) der Komponente i) können in bekannter Weise durch Alkylierung eines tertiären Amins in Gegenwart mindestens eines unverzweigten oder verzweigten Monoalkohols mit 1 bis 4 Kohlenstoffatomen, bevorzugt Ethanol, Propanol, Isopropanol, Butanol, Isobutanol und t-Butanol, besonders bevorzugt Isopropanol, hergestellt werden. Die quaternären Ammoniumverbindungen werden bevorzugt als Pellets oder besonders bevorzugt als Pulver eingesetzt. In einer bevorzugten Ausführungsform enthalten die quaternären Ammoniumverbindungen weniger als 5 Gew.-%, bevorzugt weniger als 2 Gew.-%, an Monoalkoholen. In einer weiteren bevorzugten Ausführungsform enthalten die quaternären Ammoniumverbindungen 10 bis 25 Gew.-% an Monoalkoholen. Die erfindungsgemäßen Zusammensetzungen besitzen bevorzugt einen Gesamtgehalt an unverzweigten oder verzweigten Monoalkoholen mit 1 bis 4 Kohlenstoffatomen von weniger als 5 Gew.-%, besonders bevorzugt weniger als 1 Gew.-%.
In einer ebenfalls bevorzugten Ausführungsform sind die erfindungsgemäßen Zusammensetzungen frei von unverzweigten oder verzweigten Monoalkoholen mit 1 bis 4 Kohlenstoffatomen.
Zur Einstellung des gewünschten Gehalts an Monoalkoholen in den erfindungsgemäßen Zusammensetzungen werden die Komponenten i) und/oder iv) entsprechend gewählt bzw. bemessen und/oder man entfernt die Monoalkohole teilweise oder ganz vorab aus der Komponente i).
In einer weiteren Ausführungsform werden die Monoalkohole nachträglich aus den erfindungsgemäßen Zusammensetzungen bis auf den gewünschten Restgehalt entfernt. Bevorzugt werden die Monoalkohole bei 700 bis 10 mbar, bevorzugt 400 bis 70 mbar, und 60 bis 90°C abgezogen.
Ebenso können die Monoalkohole bei Normaldruck in geeigneten Verdampfungseinrichtungen (z.B. Dünnschicht-Verdampfer) bei Temperaturen bis 120 °C abdestilliert werden.

Überraschenderweise wurde gefunden, dass die erfindungsgemäßen Zusammensetzungen auch "in situ" durch Alkylierung von
i) mindestens einem tertiären Amin NR₁R₂R₃,
   wobei R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ- steht, wobei R₅ für eine Alkyl- oder Alkenylgruppe mit 12 bis 36 Kohlenstoffatomen und n für eine Zahl von 1 bis 8 stehen,
   und R₂ und R₃ unabhängig voneinander gleich oder verschieden sein können und für -CH₃, CH₃CH₂-, CH₃CH₂CH₂-, CH₃CH₂CH₂CH₂- oder -CH₂CH₂(OH) stehen, durch
ii) mindestens ein Alkylierungsmittel, ausgewählt aus
   a) R₄X, wobei R₄ für CH₃, CH₃CH₂-, CH₃CH₂CH₂-, CH₃CH₂CH₂CH₂- oder -CH₂CH(OH)CH₂(OH) steht und X für Cl, I, Br, OSO₃H oder Methosulfat steht, und/oder
   b) Ethylenoxid und einer Säure HX, wobei X für Cl, I, Br, OSO₃H, Citrat oder Lactat steht,
   in Gegenwart von
iii) einer Mischung aus mindestens einem verzweigten Alkohol b1) mit 8 bis 36 Kohlenstoffatomen und einem unverzweigten Alkohol b2) mit 8 bis 36 Kohlenstoffatomen und
iv) mindestens einem mehrwertigen Alkohol c) und
v) gegebenenfalls mindestens einem unverzweigten oder verzweigten Monoalkohol mit 1 bis 4 Kohlenstoffatomen
   hergestellt werden können.

Bevorzugt werden bei der Umsetzung die Einsatzmengen an verzweigten Alkoholen b1), unverzweigten Alkoholen b2), mehrwertigen Alkoholen c) und gegebenenfalls unverzweigten oder verzweigten Monoalkohol mit 1 bis 4 Kohlenstoffatomen entsprechend der schon vorab beschriebenen bevorzugten Zusammensetzung der erfindungsgemäßen Zusammensetzungen gewählt. Die Gehalte können auch durch nachträgliche Zugabe oder Entfernen eingestellt werden.

In einer bevorzugten Ausführungsform werden bei der Umsetzung keine unverzweigten oder verzweigten Monoalkohole mit 1 bis 4 Kohlenstoffatomen eingesetzt.

Die nach den oben beschriebenen Verfahren hergestellten erfindungsgemäßen Zusammensetzungen werden als homogene oder inhomogene Schmelze durch Abkühlen in Pellets oder Flakes, überführt werden.

Die erfindungsgemäßen Zusammensetzungen eignen sich allgemein zur Herstellung von Mitteln, enthaltend quaternäre Ammoniumverbindungen. Besonders geeignet sind die Zusammensetzungen zur Herstellung von kosmetischen, dermatologischen und pharmazeutischen Mitteln. Insbesondere eignen sie sich für die Herstellung von Haarbehandlungsmitteln.

Gegenstand der Erfindung ist demnach auch die Verwendung der erfindungsgemäßen Zusammensetzungen zur Herstellung von Mittel, bevorzugt kosmetischen, dermatologischen und pharmazeutischen Mitteln, insbesondere Haarbehandlungsmitteln, enthaltend quaternäre Ammoniumverbindungen.

Beispiele für bevorzugte Mittel sind Shampoos, rinse-off-Haarconditionierer, Cremespülungen, Klarspülungen, Haarkuren, Haarfärbe- und Haartönungsmittel, Dauerwellmittel, Haargele, Haarconditionierer in Aerosol-, Spray- und Fluidform, 2-in-1 Duschbäder, Cremeduschbäder, Hautpflegemittel, Tagescremes, Nachtcremes, Pflegecremes, Nährcremes, Bodylotions und Salben.

Die kosmetischen, dermatologischen und pharmazeutischen Mittel enthalten die erfindungsgemäßen Zusammensetzungen, bezogen auf die fertigen Mittel, bevorzugt in Mengen von 0,1 bis 15 Gew.-%, besonders bevorzugt 1 bis 10 Gew.-%, besonders bevorzugt 1 bis 7 Gew.-%.

Die kosmetischen, dermatologischen und pharmazeutischen Mittel können als weitere Hilfs- und Zusatzstoffe alle üblichen Tenside, Ölkörper, Emulgatoren und Co-Emulgatoren, kationische Polymere, Filmbildner, Überfettungsmittel, feuchtigkeitsspendende Mittel, Stabilisatoren, biogene Wirkstoffe, Glycerin, Konservierungsmittel, Perlglanzmittel, Farb- und Duftstoffe, Lösungsmittel, Hydrotrope, Trübungsmittel, Verdickungsmittel, Dispergiermittel, Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme, Trägersubstanzen, Antioxidation, UV-Lichtschutzfilter, Pigmente und Metalloxide, sowie antimikrobiell wirkende Agentien enthalten.

Als Tenside können anionische, kationische, nichtionische, amphotere und/oder zwitterionische Tenside verwendet werden. Bevorzugte nichtionische Tenside enthalten als hydrophile Gruppe eine Polyolgruppe, eine Polyolalkenylethergruppe oder eine Kombination aus Polyol- und Polyglykolethergruppen. Bevorzugt sind Anlagerungsprodukte aus von 2 bis 30 Mol Ethylenoxid, 2 bis 30 Mol Ethylenoxid zusammen mit bis 5 Mol Propylenoxid oder von bis 5 Mol Propylenoxid an lineare Fettalkohole mit 8 bis 22 C-Atomen und Alkylphenolen mit 8 bis 15 C-Atomen in der Alkylgruppe, (C₁₂-C₁₉)-Fettsäuremono- und diester von Anlagerungsprodukten von 1 bis 30 Mol Ethylenoxid an Glycerin, Glycerinmono- und -diester und Sorbitanmono- und -diester von gesättigten und ungesättigten (C₈-C₁₈)-Fettsäuren und deren Ethylenoxidanlagerungsprodukte, (C₈-C₁₈)-A)kylmono- und -oligoglykoside und deren ethoxylierte Analoga, Anlagerungsprodukte von 10 bis 60 Mol Ethylenoxid an Ricinusöl und gehärtetes Ricinusöl, ethoxylierte und nicht ethoxylierte Mono-, Di- und Trialkylmonophosphor-säureester, insbesondere Mono-, Di- und Tri-(Lauryltetraglycolether)-o-Phosphor-säureester und Mono-, Di- und Tri-(Cetyltetraglycolether)-o-Phosphorsäureester.

Bevorzugte amphotere Tenside tragen eine (C₈-C₁₈)-Alkyl- oder -Acylgruppe und mindestens eine freie Aminogruppe und mindestens eine -COOH- oder -SO₃H-Gruppe. Bevorzugt sind N-Acylglycine, N-Alkylpropionsäure, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 18 C-Atomen in der Alkylgruppe. Besonders bevorzugte sind N-Kokosalkylaminopropionat, Kokosacylaminoethylaminopropionat und (C₁₂-C₁₈)-Alkylsarcosine.

Besonders geeignete zwitterionische Tenside sind Betaine, wie beispielsweise die N-Alkyl-N,N-dimethylammoniumglycinate, z.B. Kokosalkyldimethylammoniumglycinate, N-Acyl-aminopropyl-N-N-dimethylammoniumglycinate, z.B. Kokosacylaminopropyl-dimethylammoniumglycinat, 2-Alkyl-3-carboxymethyl-3-hydroxyethylimidazoline mit jeweils 8 bis 18 C-Atomen in der Alkyl- oder Acylgruppe und das Kokosacylamino-ethylhydroxyethylcarboxymethylglycinat.

Bevorzugt enthalten die Mittel Tensid-Mischungen, besonders bevorzugt sind Mischungen aus nichtionischen und zwitterionischen oder amphoteren Tensiden in einem Gewichtsverhältnis von 5 zu 1 bis 1 zu 5 oder Mischungen aus nichtionogenen Tensiden und beliebigen Mischungen aus zwitterionischen und amphoteren Tensiden in einem Gewichtsverhältnis von 5 zu 1 bis 1 zu 5.

Als Ölkörper eignen sich alle bekannten Öle, Fette und Wachse mineralischer, tierischer, pflanzlicher und synthetischer Herkunft. Bevorzugt sind als Öl- und Fettkomponenten Diallylether mit insgesamt 12 bis 24 C-Atomen, Fettsäureester mit insgesamt 12 bis 26 C-Atomen, flüssige Kohlenwasserstoffe mit 10 bis 32 C-Atomen und Gemische davon. Geeignete Fettsäureester sind z.B. Methylpalmitat, Ethyloleat, Isopropylmyristat, n-Hexyllaurat, n-Butylstearat und Cetyl-/ Stearylisononanoat. Besonders bevorzugt sind Paraffinöle, Vaseline, Pflanzenöle, synthetische Triglyceride wie z.B. Glyceryltricaprylat, sowie Silikonöle.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden. Als Konsistenzgeber kommen Fettalkohole mit 12 bis 22, vorzugsweise 12 bis 18 Kohlenstoffatomen sowie Partialglyceride in Betracht.

Als weitere Verdickungsmittel können Polysaccharide, insbesondere Xantham-Gum, Guar-Guar, Agar-Agar, Alginate, Carboxymethylcellulose, Hydroxyethylcellulose, höhermolekulare Polyethylenglykolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylpropan, Fettalkoholethoxylate oder Alkyloligoglucoside, sowie Elektrolyte wie Kochsalz und Ammoniumchlorid eingesetzt werden.

Geeignete Siliconverbindungen sind beispielsweise Dimethylpolysiloxan, Methylphenylpolysiloxane, cyclische Silicone sowie amino-, fettsäure-, alkohol-, polyether-, epoxy-, fluor- und/ oder alkylmodifizierte Siliconverbindungen, die bei Raumtemperatur sowohl flüssig als auch harzförmig vorliegen können.

Unter biogenen Wirkstoffen sind beispielsweise Bisabolol® , Allantoin® , Phytantriol® , Panthenol® , AHA-Säuren, Pflanzenextrakte und Vitaminkomplexe zu verstehen.

Als Antischuppenmittel können Climbazol® , Octopirox® , Oxiconazol® und Zinkpyrethion® eingesetzt werden.

Gebräuchliche Filmbildner sind Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Zur Verbesserung des Fließverhaltens können ferner Hydrotrope wie beispielsweise Ethanol, Isopropylalkohol, Propylenglykol oder Glucose eingesetzt werden.

Als Konservierungsmittel eignen sich beispielsweise Phenoxyethanol, Formaldehydlösung, Parabene, Pentandiol und Sorbinsäure.

Als feuchtigkeitsspendende Substanz stehen beispielsweise Isopropylpalmitat, Glycerin und/oder Sorbitol zu Verfügung.

Der Gesamtanteil an Hilfs- und Zusatzstoffen in den Mitteln beträgt bevorzugt 1 bis 50 Gew.-%, besonders bevorzugt 5 bis 40 Gew.-%.

Die nachfolgenden Beispiele dienen der Erläuterung der Erfindung, ohne sie jedoch einzuschränken.

### Beispiele

### a) quaternäre Ammoniumverbindungen (Behenyltrimethylammoniumchlorid):

Im Lösemittel Isopropanol hergestelltes Behenyltrimethylammoniumchlorid wurden in Form von Pellets zur Entfernung des Lösemittels in einem Vakuumofen 14 Stunden bei einer Temperatur von 74°C und 200 mbar Druck bis zur Gewichtskonstanz getrocknet. Die so getrockneten Pellets wurden zu einem Pulver gemahlen und gesiebt (Siebweite: 630 µm).

### b) Herstellung der erfindungsgemäßen Zusammensetzungen:

Das in a) erhaltene Behenyltrimethylammoniumchlorid-Pulver wurde mit einer Mischung aus verzweigten Alkoholen b1) und unverzweigten Alkoholen b2) und einem mehrwertigen Alkohol c) vermischt. Anschließend wurden die Mischungen in einer verschlossenen Pulverflasche in einem Trockenschrank unter Erwärmen und mehrmaligem Rühren homogenisiert bis das Pulver vollständig gelöst war. Durch langsames Absenken der Temperatur im Trockenschrank wurden die Stockpunkte ermittelt. Die Quat-Aktivität wurde durch Kation-Titration bestimmt.

### Beispiel 1:

6,7 g getrocknetes und gesiebtes Behenyltrimethylammoniumchlorid wurde in einer 50 ml-Pulverflasche aus Glas vorgelegt und mit 3,0 g ® LIAL 145 (Fa. Sasol) und 0,3 g einer 87 gew.-%igen wässrigen Glycerinlösung versetzt. Nach Verschließen der Flasche wurde die Mischung 12 Stunden auf ca. 100°C erhitzt. Während dieser Zeit wurde die Probe mit einem Spatel mehrmals gerührt, um die Mischung zu homogenisieren. Man erhielt eine klare, leicht gelbliche Lösung, die bei 80-84°C erstarrte und bei 90 °C wieder vollständig geschmolzen vorlag. Bei 25 °C war die Zusammensetzung wachsartig fest. Der Quat-Aktivgehalt betrug 67 Gew.-%.

### Beispiel 2:

6,5 g getrocknetes und gesiebtes Behenyltrimethylammoniumchlorid wurde in einer 50 ml-Pulverflasche aus Glas vorgelegt und mit 2,9 g ® LIAL 145 (Fa. Sasol) und 0,6 g Propylenglykol versetzt. Nach Verschließen der Flasche wurde die Mischung 4 Stunden auf ca. 100°C und noch weitere 12 Stunden bei 90°C erhitzt. Während dieser Zeit wurde die Probe mit einem Spatel mehrmals gerührt, um die Mischung zu homogenisieren. Man erhielt eine klare, leicht gelbliche Lösung, die bei 76-81 °C langsam erstarrte und bei 90°C wieder vollständig klar geschmolzen vorlag. Bei 25°C war die Zusammensetzung wachsartig fest. Der Quat-Aktivgehalt betrug 65 Gew.-%.

### Beispiel 3:

7,0 g getrocknetes und gesiebtes Behenyltrimethylammoniumchlorid wurde in einer 50 ml-Pulverflasche aus Glas vorgelegt und mit 2,4 g ® LIAL 145 (Fa. Sasol) und 0,6 g Propylenglykol versetzt. Nach Verschließen der Flasche wurde die Mischung 4 Stunden auf ca. 100°C und noch weitere 12 Stunden bei 90°C erhitzt. Während dieser Zeit wurde die Probe mit einem Spatel mehrmals gerührt, um die Mischung zu homogenisieren. Man erhielt eine klare, leicht gelbliche Lösung, die bei 80-84°C langsam erstarrte und bei 90°C wieder vollständig klar geschmolzen vorlag. Bei 25°C war die Zusammensetzung wachsartig fest. Der Quat-Aktivgehalt betrug 70 Gew.-%.

### Beispiel 4:

6,7 g getrocknetes und gesiebtes Behenyltrimethylammoniumchlorid wurde in einer 50 ml-Pulverflasche aus Glas vorgelegt und mit 2,8 g ® LIAL 145 (Fa. Sasol) und 0,5 g 1,3-Butandiol versetzt. Nach Verschließen der Flasche wurde die Mischung 16 Stunden auf ca. 100°C erhitzt. Während dieser Zeit wurde die Probe mit einem Spatel mehrmals gerührt, um die Mischung zu homogenisieren. Man erhielt eine klare, leicht gelbliche Lösung, die bei 95-97°C langsam erstarrte und bei 100°C wieder vollständig klar geschmolzen vorlag. Bei 25°C war die Zusammensetzung wachsartig fest. Der Quat-Aktivgehalt betrug 67 Gew.-%.

### Beispiel 5:

4,88 g getrocknetes und gesiebtes Behenyltrimethylammoniumchlorid wurde in einer 50 ml-Pulverflasche aus Glas vorgelegt und mit 4.61 g ® LIAL 145 (Fa. Sasol) und 0,51 g 1,3-Butandiol versetzt. Nach Verschließen der Flasche wurde die Mischung 4 Stunden auf ca. 100°C und noch weitere 12 Stunden bei 90°C erhitzt. Während dieser Zeit wurde die Probe mit einem Spatel mehrmals gerührt, um die Mischung zu homogenisieren. Man erhielt eine klare, leicht gelbliche Lösung, die bei 68-72°C langsam erstarrte und bei 90°C wieder vollständig klar geschmolzen vorlag. Bei 25°C war die Zusammensetzung wachsartig fest. Der Quat-Aktivgehalt betrug 48 Gew.-%.

### Vergleichsbeispiel 4a:

6,7 g getrocknetes und gesiebtes Behenyltrimethylammoniumchlorid wurde in einer 50 ml-Pulverflasche aus Glas vorgelegt und mit 2,8 g Cetylakohol und 0,5 g 1,3-Butandiol versetzt. Nach Verschließen der Flasche wurde die Mischung 16 Stunden auf ca. 100°C erhitzt. Während dieser Zeit wurde die Probe mit einem Spatel mehrmals gerührt. Man erhielt eine zweiphasige Zusammensetzung deren Stockpunkt nicht bestimmt werden konnte.

### Vergleichsbeispiel 5a:

4,88 g getrocknetes und gesiebtes Behenyltrimethylammoniumchlorid wurde in einer 50 ml-Pulverflasche aus Glas vorgelegt und mit 4,61 g Cetylalkohol und 0,51 g 1,3-Butandiol versetzt. Nach Verschließen der Flasche wurde die Mischung 4 Stunden auf ca. 100°C und noch weitere 12 Stunden bei 90°C erhitzt. Während dieser Zeit wurde die Probe mit einem Spatel mehrmals gerührt, um die Mischung zu homogenisieren. Man erhielt eine klare Lösung, die bei 84-86°C langsam erstarrte und bei 90°C wieder vollständig klar geschmolzen vorlag.

Die Beispiele 6 bis 8 zeigen Formulierungsbeispiele. Die erfindungsgemäßen Zusammensetzungen wurden durch Auftropfen auf eine kalte Metallplatte pelletiert und anschließend in die Formulierungen eingearbeitet.

### Beispiel 6: Cremespülung

| | | |
|---|---|---|
| A. | erfindungsgemäße Zusammensetzung Bsp. 1 | 2,2 Gew.-% |
| | ® HOSTAPHAT KL 340 D (Trilaureth-4 Phosphate, Fa. Clariant) | 1,5 Gew.-% |
| | Cetylalkohol | 3 Gew.% |
| | Paraffinöl | 1 Gew.-% |
| B | Wasser | 92,3 Gew.-% |
| C | Citronensäure | q.s. |

### Herstellung:

- I): A bei 75°C aufschmelzen
- II): B auf 75°C erwärmen
- III): B in A einrühren und die Mischung bis zum Abkühlen weiterrühren
- IV): pH-Wert mittels C auf pH = 4 einstellen

### Beispiel 7: O/W-Handcreme

| | | |
|---|---|---|
| A | Zusammensetzung aus Bsp. 1 | 3 Gew.-% |
| | ® Hostacerin DGSB (PEG-4 Polyglyceryl-2 Stearate, Fa. Clariant) | 6 Gew.-% |
| | Paraffinöl, hochviskos | 10 Gew.-% |
| | Isopropylpalmitat | 10 Gew.-% |
| B | Wasser | 70,6 Gew.-% |
| | Konservierungsmittel | q.s. |
| C | Parfüm - | 0,4 Gew.-% |

### Herstellung:

- I): A bei 80°C aufschmelzen
- II): B auf 80°C erhitzen
- III): B in A einrühren und die Mischung bis zum Abkühlen weiterrühren
- IV): C bei 35°C zu III gegeben.

### Beispiel 8: Haarconditionierer mit Perlglanzeffekt

| | | |
|---|---|---|
| A | Zusammensetzung aus Bsp. 1 | 2,2 Gew.-% |
| | ® Genamin KSL (PEG- 5 Stearyl Ammonium Lactate, Fa. Clariant) | 9 Gew.-% |
| | ® Hostaphat KL 340 D (Trilaureth- 4 Phosphate, Fa. Clariant) | 1,5 Gew.-% |
| | Jojoba Öl | 1 Gew.-% |
| B | ® Tylose H 100 000 YP2 (Hydroxyethylcellulose, Fa. Clariant) | 1,5 Gew.% |
| C | Wasser | ad 100 % |
| D | Parfüm | 0,5 Gew.-% |
| | Panthenol | 0,5 Gew.-% |
| | Genapol PDC (Fa. Clariant) | 4 Gew.-% |
| | (Glycol Distearate, Laureth-4-, Cocamidopropyl Betaine, Glimmer, Titandioxid) | |
| E | Citronensäure | q.s. |

### Herstellung:

- I): Komponenten A auf 75°C erwärmen
- II): B gut in C lösen und auf 75°C erwärmen
- III): II unter Rühren zu I geben
- IV): Unter Rühren abkühlen lassen und bei 30°C nacheinander die Komponenten D in III geben
- V): pH-Wert mittels E auf pH = 4 einstellen

## Patentansprüche

1. Zusammensetzungen, enthaltend, bezogen auf die fertigen Zusammensetzungen,
a) 30 bis 90 Gew.-% mindestens einer quaternären Ammoniumverbindung gemäß Formel (1) 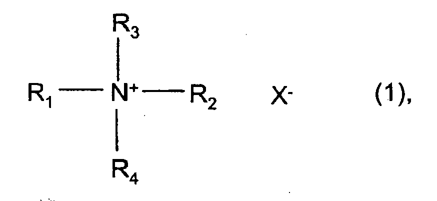 wobei
R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙsteht, wobei R₅ für eine Alkyl- oder Alkenylgruppe mit 12 bis 36 Kohlenstoffatomen und n für eine Zahl von 1 bis 8 stehen, und
R₂, R₃ und R₄ unabhängig voneinander gleich oder verschieden sein können und für eine -CH₃, CH₃CH₂-, CH₃CH₂CH₂-, CH₃CH₂CH₂CH₂-, -CH₂CH₂OH oder -CH₂CH(OH)CH₂OH-Gruppe stehen
und
X⁻ für ein Anion steht,
und
b) eine Mischung aus mindestens einem verzweigten Alkohol b1) mit 8 bis 36 Kohlenstoffatomen und mindestens einem unverzweigten Alkohol b2) mit 8 bis 36 Kohlenstoffatomen, wobei das Gewichtsverhältnis von verzweigten Alkoholen b1) zu unverzweigten Alkoholen b2) 90:10 bis 30:70 beträgt, und
c) mindestens einen mehrwertigen Alkohol mit 2 bis 6 Kohlenstoffatomen **dadurch gekennzeichnet, dass** sie in Form von Pellets oder Flakes vorliegen.

2. Zusammensetzungen nach Anspruch 1, **dadurch gekennzeichnet, dass** der Gehalt an quaternären Ammoniumverbindungen a), bezogen auf die fertigen Zusammensetzungen, 50 bis 90 Gew.-% beträgt.

3. Zusammensetzungen nach Anspruch 1 oder 2, **dadurch gekennzeichnet ,dass** es sich bei den quaternären Ammoniumverbindungen a) um (C₁₂-C₃₆)-, bevorzugt (C₁₄-C₃₀)-, besonders bevorzugt (C₁₆-C₂₄)-Alkyltrimethylammoniumverbindungen, handelt.

4. Zusammensetzungen nach mindestens einem der Ansprüche 1 bis 3, **dadurch gekennzeichnet, dass** es sich beim Anion X⁻ in Formel (1) um Chlorid, lodid, Bromid, Methosulfat, Hydrogensulfat, Lactat und/oder Citrat, besonders bevorzugt Chlorid und/oder Methosulfat, handelt.

5. Zusammensetzungen nach mindestens einem der Ansprüche 1 bis 4, **dadurch gekennzeichnet, dass** es sich bei der quaternären Ammoniumverbindung a) um Behenyltrimethylammoniumchlorid handelt.

6. Zusammensetzungen nach mindestens einem der Ansprüche 1 bis 5, **dadurch gekennzeichnet, dass** der Gehalt an Alkoholen b), bezogen auf die fertigen Zusammensetzungen, 10 bis 60 Gew.-%, bevorzugt 10 bis 50 Gew.-%, besonders bevorzugt 20 bis 40 Gew.-%, beträgt.

7. Zusammensetzungen nach mindestens einem der Ansprüche 1 bis 6, **dadurch gekennzeichnet, dass** das Gewichtsverhältnis von verzweigten Alkoholen b1) zu unverzweigten Alkoholen b2) 70 : 30 bis 30 : 70, bevorzugt 60 : 40 bis 40 : 60 beträgt.

8. Zusammensetzungen nach mindestens einem der Ansprüche 1 bis 7, **dadurch gekennzeichnet, dass** die verzweigten Alkohole b1) und die unverzweigten Alkohole b2) jeweils 10 bis 24, bevorzugt 12 bis 18, besonders bevorzugt 14 bis 16, Kohlenstoffatome besitzen.

9. Zusammensetzungen nach mindestens einem der Ansprüche 1 bis 8, **dadurch gekennzeichnet, dass** es sich bei den verzweigten Alkohole b1) um Guerbetalkohole, 2-Methyl-1-decanol, 2-Ethyl-1-nonanol, 2-Propyl-1-octanol, 2-Butyloctanol, 2-Butyl-decanol, 2-Hexyloctanol, 2-Hexyldecanol, 2-Octyldecanol, 2-Hexyldodecanol, 2-Octyldodecanol, 2-Decyltetradecanol, 2-Butyl-1-heptanol, 2-Dodecylhexadecanol, Tetradecyloctadecanol, 2-Tetradecyleicosanol, 2-Hexadecyloctadecanol, 2-Hexadecyleicosanol, 2-Methyltridecanol, 2-Ethyldodecanol, 2-Propylundecanol, 2-Pentylnonanol, 2-Heptylheptanol, 2-Methyltetradecanol, 2-Ethyltridecanol, 2-Propyldodecanol, 2-Butylundecanol, 2-Pentyldecanol, 2-Hexylnonanol, 2-Heptyloctanol, Isotridecylalkohol und/oder deren Mischungen handelt.

10. Zusammensetzungen nach mindestens einem der Ansprüche 1 bis 9, **dadurch gekennzeichnet, dass** es sich bei den unverzweigten Alkohole b2) um Fettalkohole, bevorzugt Lauryl-, Myristyl-, Cetyl-, Stearyl, Eicosanyl- und/oder Behenylalkohol, und/oder Nonanol, Undecanol, Tridecanol, Pentadecanol und/oder Heptadecanol handelt.

11. Zusammensetzungen nach mindestens einem der Ansprüche 1 bis 10, **dadurch gekennzeichnet, dass** der Gehalt an mehrwertigen Alkoholen c), bezogen auf die fertigen Zusammensetzungen, 0.5 bis 20 Gew.-%, bevorzugt 0.5 bis 10 Gew.-%, beträgt.

12. Zusammensetzungen nach mindestens einem der Ansprüche 1 bis 11, **dadurch gekennzeichnet, dass** es sich bei den mehrwertigen Alkoholen c) um Glykole, bevorzugt Ethylenglykol und Propylenglykol, Butandiole, bevorzugt 1,3-Butandiol, Glycerin, Thioglycerin, Sorbitol, Xylitol und Mannitol handelt.

13. Zusammensetzungen nach mindestens einem der Ansprüche 1 bis 12, **dadurch gekennzeichnet, dass** sie unverzweigte oder verzweigte Monoalkohole mit 1 bis 4 Kohlenstoffatomen, bevorzugt Ethanol, Propanol, Isopropanol, Butanol, Isobutanol und/oder t-Butanol, besonders bevorzugt Isopropanol, enthalten.

14. Zusammensetzungen nach Anspruch 13, **dadurch gekennzeichnet, dass** sie, bezogen auf die fertigen Zusammensetzungen, weniger als 5 Gew.-%, bevorzugt weniger als 1 Gew.-%, an unverzweigten oder verzweigten Monoalkoholen mit 1 bis 4 Kohlenstoffatomen enthalten.

15. Zusammensetzungen nach mindestens einem der Ansprüchen 1 bis 14, **dadurch gekennzeichnet, dass** sie einen Stockpunkt unterhalb 100°C, bevorzugt unterhalb 95°C, besonders bevorzugt unterhalb 90°C, insbesondere unterhalb 85°C, besitzen.

16. Zusammensetzungen nach mindestens einem der Ansprüche 1 bis 15, **dadurch gekennzeichnet, dass** sie einen Flammpunkt oberhalb 80°C, bevorzugt oberhalb 100°C, besitzen.

17. Verfahren zur Herstellung von Zusammensetzungen gemäß mindestens einem der Ansprüche 1 bis 16, **dadurch gekennzeichnet, dass** eine Mischung hergestellt wird, enthaltend
i) mindestens eine quaternäre Ammoniumverbindung a), gegebenenfalls enthaltend einen verzweigten oder unverzweigten Monoalkohol mit 1 bis 4 Kohlenstoffatomen,
ii) eine Mischung aus mindestens einem verzweigten Alkohol b1) mit 8-36 Kohlenstoffatomen und mindestens einem unverzweigten Alkohol b2) mit 8 bis 36 Kohlenstoffatomen,
iii) mindestens einen mehrwertigen Alkohol c) und
iv) gegebenenfalls mindestens einen unverzweigten oder verzweigten Monoalkohol mit 1 bis 4 Kohlenstoffatomen.

18. Verfahren zur Herstellung einer Zusammensetzung gemäß mindestens einem der Ansprüche 1 bis 16, **dadurch gekennzeichnet, dass**
i) mindestens ein tertiäres Amin NR₁R₂R₃, wobei R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ- steht, wobei R₅ für eine Alkyl- oder Alkenylgruppe mit 12 bis 36 Kohlenstoffatomen und n für eine Zahl von 1 bis 8 stehen, und R₂ und R₃ unabhängig voneinander gleich oder verschieden sein können und für CH₃-, CH₃CH₂-, CH₃CH₂CH₂-, CH₃CH₂CH₂CH₂- oder -CH₂CH₂(OH) stehen,
durch
ii) mindestens ein Alkylierungsmittel, ausgewählt aus
a) R₄X, wobei R₄ fürCH₃-, CH₃CH₂-, CH₃CH₂CH₂-, CH₃CH₂CH₂CH₂- oder -CH₂CH(OH)CH₂(OH) steht und X für Cl, I, Br, OSO₃H oder Methosulfat steht, und/oder
b) ethylenoxid und einer Saure HX, wobei X für Cl, I, Br, OSO₃H, Citrat oder Lactat steht,
in Gegenwart von
iii) einer Mischung aus mindestens einem verzweigten Alkohol b1) mit 8-36 Koklenstoffatomen und einem unverzweigten Alkohol b2) mit 8 bis 36 Kohlenstoffatomen und
iv) von mindestens einem mehrwertigen Alkohol c) und
v) gegebenenfalls mindestens einem unverzweigten oder verzweigten Monoalkohol mit 1 bis 4 Kohlenstoffatomen
alkyliert wird.

19. Verwendung einer Zusammensetzung gemäß mindestens einem der Ansprüche 1 bis 16 zur Herstellung von kosmetischen, dermatologischen und pharmazeutischen Mitteln, bevorzugt Haarbehandlungsmitteln.

20. Verwendung nach Anspruch 19, **dadurch gekennzeichnet, dass** es sich bei den Haarbehandlungsmitteln um Shampoos, rinse-off-Haarconditioner, Cremespülungen, Klarspülungen, Haarkuren, Haarfärbe- und Haartönungsmittel, Dauerwellmittel, Haargele und Haarconditioner in Aerosol-, Spray- und Fluidform handelt.

## Claims

1. A composition comprising, based on the finished composition
a) 30 to 90% by weight of at least one quaternary ammonium compound according to formula (1) 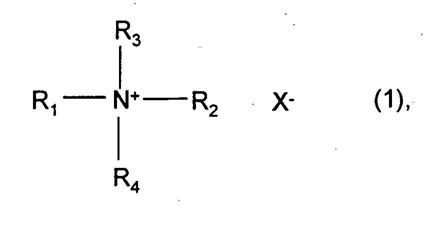 where
R₁ is an unbranched or branched alkyl or alkenyl group having 12 to 36 carbon atoms, a group R₅CONH(CH₂)ₙ- or a group R₅COO(CH₂)ₙ-, where R₅ is an alkyl or alkenyl group having 12 to 36 carbon atoms and n is a number from 1 to 8, and
R₂, R₃ and R₄, independently of one another, may be identical or different and are a -CH₃, CH₃CH₂-, CH₃CH₂CH₂-, CH₃CH₂CH₂CH₂-, CH₂CH₂OH or CH₂CH(OH)CH₂OH group
and
X⁻ is an anion,
and
b) a mixture of at least one branched alcohol b1) having 8 to 36 carbon atoms and at least one unbranched alcohol b2) having 8 to 36 carbon atoms, where the weight ratio of branched alcohols b1) to unbranched alcohols b2) is 90:10 to 30:70 and
c) at least one polyhydric alcohol having 2 to 6 carbon atoms, said composition being in the form of pellets or flakes.

2. The composition as claimed in claim 1, wherein the content of quaternary ammonium compounds a), based on the finished composition, is 50 to 90% by weight.

3. The composition as claimed in claim 1 or 2, wherein the quaternary ammonium compounds a) are (C₁₂-C₃₆)-, preferably (C₁₄-C₃₀)-, particularly preferably (C₁₆-C₂₄)-alkyltrimethylammonium compounds.

4. The composition as claimed in at least one of claims 1 to 3, wherein the anion X- in formula (1) is chloride, iodide, bromide, methosulfate, hydrogensulfate, lactate and/or citrate, particularly preferably chloride and/or methosulfate.

5. The composition as claimed in at least one of claims 1 to 4, wherein the quaternary ammonium compound a) is behenyltrimethylammonium chloride.

6. The composition as claimed in at least one of claims 1 to 5, wherein the content of alcohols b), based on the finished composition, is 10 to 60% by weight, preferably 10 to 50% by weight, particularly preferably 20 to 40% by weight.

7. The composition as claimed in at least one of claims 1 to 6, wherein the weight ratio of branched alcohols b1) to unbranched alcohols b2) is 70:30 to 30:70, preferably 60:40 to 40:60.

8. The composition as claimed in at least one of claims 1 to 7, wherein the branched alcohols b1) and the unbranched alcohols b2) each have 10 to 24, preferably 12 to 18, particularly preferably 14 to 16, carbon atoms.

9. The composition as claimed in at least one of claims 1 to 8, wherein the branched alcohols b1) are Guerbet alcohols, 2-methyl-1-decanol, 2-ethyl-1-nonanol, 2-propyl-1-octanol, 2-butyloctanol, 2-butyldecanol, 2-hexyloctanol, 2-hexyldecanol, 2-octyldecanol, 2-hexyldodecanol, 2-octyldodecanol, 2-decyltetradecanol, 2-butyl-1-heptanol, 2-dodecylhexadecanol, tetradecyloctadecanol, 2-tetradecyleicosanol, 2-hexadecyloctadecanol, 2-hexadecyleicosanol, 2-methyltridecanol, 2-ethyldodecanol, 2-propylundecanol, 2-pentylnonanol, 2-heptylheptanol, 2-methyltetradecanol, 2-ethyltridecanol, 2-propyldodecanol, 2-butylundecanol, 2-pentyldecanol, 2-hexylnonanol, 2-heptyloctanol, isotridecyl alcohol and/or mixtures thereof.

10. The composition as claimed in at least one of claims 1 to 9, wherein the unbranched alcohols b2) are fatty alcohols, preferably lauryl alcohol, myristyl alcohol, cetyl alcohol, stearyl alcohol, eicosanyl alcohol and/or behenyl alcohol, and/or nonanol, undecanol, tridecanol, pentadecanol and/or heptadecanol.

11. The composition as claimed in at least one of claims 1 to 10, wherein the content of polyhydric alcohols c), based on the finished composition, is from 0.5 to 20% by weight, preferably 0.5 to 10% by weight.

12. The composition as claimed in at least one of claims 1 to 11, wherein the polyhydric alcohols c) are glycols, preferably ethylene glycol and propylene glycol, butanediols, preferably 1,3-butanediol, glycerol, thioglycerol, sorbitol, xylitol and mannitol.

13. The composition as claimed in at least one of claims 1 to 12, which comprises unbranched or branched monoalcohols having 1 to 4 carbon atoms, preferably ethanol, propanol, isopropanol, butanol, isobutanol and/or t-butanol, particularly preferably isopropanol.

14. The composition as claimed in claim 13, which comprises, based on the finished composition, less than 5% by weight, preferably less than 1% by weight, of unbranched or branched monoalcohols having 1 to 4 carbon atoms.

15. The composition as claimed in at least one of claims 1 to 14, which has a setting point below 100°C, preferably below 95°C, particularly preferably below 90°C, especially preferably below 85°C.

16. The composition as claimed in at least one of claims 1 to 15, which has a flash point above 80°C, preferably above 100°C.

17. A process for the preparation of compositions as claimed in at least one of claims 1 to 16, which comprises preparing a mixture comprising
i) at least one quaternary ammonium compound a), optionally containing a branched or unbranched monoalcohol having 1 to 4 carbon atoms,
ii) a mixture of at least one branched alcohol b1) having 8 to 36 carbon atoms and at least one unbranched alcohol b2) having 8 to 36 carbon atoms,
iii) at least one polyhydric alcohol c) and
iv) optionally at least one unbranched or branched monoalcohol having 1 to 4 carbon atoms.

18. A process for the preparation of a composition as claimed in at least one of claims 1 to 16, which comprises alkylating
i) at least one tertiary amine NR₁R₂R₃, where
R₁ is an unbranched or branched alkyl or alkenyl group having 12 to 36 carbon atoms, a group R₅CONH(CH₂)ₙ- or a group R₅COO(CH₂)ₙ-, where R₅ is an alkyl or alkenyl group having 12 to 36 carbon atoms and n is a number from 1 to 8,
and R₂ and R₃, independently of one another, may be identical or different and are -CH₃, CH₃CH₂-, CH₃CH₂CH₂-, CH₃CH₂CH₂CH₂- or -CH₂CH₂(OH),
by
ii) at least one alkylating agent chosen from
a) R₄X, where R₄ is -CH₃-, CH₃CH₂-, CH₃CH₂CH₂-, CH₃CH₂CH₂CH₂- or -CH₂CH(OH)CH₂(OH), and X is Cl, l, Br, OSO₃H or methosulfate, and/or
b) ethylene oxide and an acid HX, where X is .CI, I, Br, OSO₃H, citrate or lactate,
in the presence of
iii) a mixture of at least one branched alcohol b1) having 8 to 36 carbon atoms and one unbranched alcohol b2) having 8 to 36 carbon atoms and
iv) of at least one polyhydric alcohol c) and
v) optionally at least one unbranched or branched monoalcohol having 1 to 4 carbon atoms.

19. The use of a composition as claimed in at least one of claims 1 to 16 for the preparation of cosmetic, dermatological and pharmaceutical compositions, preferably hair-treatment compositions.

20. The use as claimed in claim 19, wherein the hair-treatment compositions are shampoos, rinse-off hair conditioners, cream rinses, clear rinses, hair cures, hair colorants and hair tints, permanent waving compositions, hair gels and hair conditioners in aerosol form, spray form and fluid form.

## Revendications

1. Compositions comprenant, par rapport aux compositions finies,
a) 30% à 90% en poids d'au moins un composé ammonium quaternaire de formule (1) 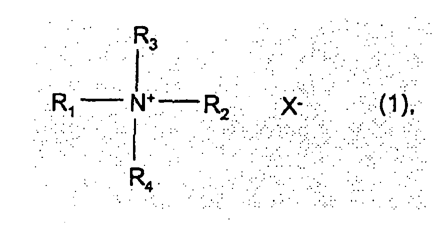 dans laquelle
R₁ représente un groupe alkyle ou alcényle non ramifié ou ramifié contenant 12 à 36 atomes de carbone, un groupe R₅CONH(CH₂)ₙ- ou un groupe R₅COO(CH₂)ₙ-, dans lesquels R₅ représente un groupe alkyle ou alcényle contenant 12 à 36 atomes de carbone et n représente un nombre allant de 1 à 8 et
R₂, R₃ et R₄ peuvent être, indépendamment les uns des autres, identiques ou différents et représentent un groupe -CH₃, CH₃CH₂-, CH₃CH₂CH₂-, CH₃CH₂CH₂CH₂-, CH₂CH₂OH ou -CH₂CH(OH)CH₂OH
et
X⁻ représente un anion
et
b) un mélange d'au moins un alcool ramifié b1) contenant 8 à 36 atomes de carbone et d'au moins un alcool non ramifié b2) contenant 8 à 36 atomes de carbone, dans lequel le rapport pondéral de l'alcool ramifié b1) à l'alcool non ramifié b2) va de 90:10 à 30:70, et
c) au moins un polyol contenant 2 à 6 atomes de carbone,
**caractérisées en ce qu'**elle se présentent sous forme de granulés ou de flocons.

2. Compositions selon la revendication 1, **caractérisées en ce que** la teneur en composés ammonium quaternaire a), par rapport aux compositions finies, va de 50% à 90% en poids.

3. Compositions selon la revendication 1 ou 2, **caractérisées en ce que** les composés ammonium quaternaire a) sont des composés (alkyle en C₁₂ à C₃₆)triméthylammonium, de préférence (alkyle en C₁₄ à C₃₀)triméthylammonium, de manière plus particulièrement préférée (alkyle en C₁₆ à C₂₄)triméthylammonium.

4. Compositions selon au moins l'une quelconque des revendications 1 à 3, **caractérisées en ce que** l'anion X- dans la formule (1) est un anion chlorure, iodure, bromure, méthosulfate, hydrogénosulfate, lactate et/ou citrate, de manière plus particulièrement préférée chlorure et/ou méthosulfate.

5. Compositions selon au moins l'une quelconque des revendications 1 à 4, **caractérisées en ce que** le composé ammonium quaternaire a) est le chlorure de béhényltriméthylammonium.

6. Compositions selon au moins l'une quelconque des revendications 1 à 5, **caractérisées en ce que** la teneur en alcools b), par rapport aux compositions finïes, va de 10% à 60% en poids, de préférence de.10.% à 50% en poids et de manière plus particulièrement préférée de 20% à 40% en poids.

7. Compositions selon au moins l'une quelconque des revendications 1 à 6, **caractérisées en ce que** le rapport pondéral de l'alcool ramifié b1) à l'alcool non ramifié b2) va de 70:30 à 30:70, de préférence de 60:40 à 40:60.

8. Compositions selon au moins l'une quelconque des revendications 1 à 7, **caractérisées en ce que** les alcools ramifiés b1) et les alcools non ramifiés b2) contiennent chacun 10 à 24, de préférence 12 à 18, et de manière plus particulièrement préférée 14 à 16 atomes de carbone.

9. Compositions selon au moins l'une quelconque des revendications 1 à 8, **caractérisées en ce que** les alcools ramifiés b1) sont des alcools de Guerbet, le 2-méthyl-1-décanol, le 2-éthyl-1-nonanol, le 2-propyl-1-octanol, le 2-butyloctanol, le 2-butyldécanol, le 2-hexyloctanol, le 2-hexyldécanol, le 2-octyldécanol, le 2-hexyldodécanol, le 2-octyldodécanol, le 2-décyltétradécanol, le 2-butyl-1-heptanol, le 2-dodécylhexadécanol, le tëtradécyloctadécanôl, le 2-tétadécyleicosanol, le 2-hexadécyloctadécanol, le 2-hexadécyleicosanol, le 2-méthyltridécanol, le 2-éthyldodécanol, le 2-propylundécanol, le 2-pentylnônanol, le 2-heptylheptanol, le 2-méthyltétradécanol, le 2-éthyltrïdécanol, le 2-propyldodécanol, le 2-butylundécanol, le 2-pentyldécanol, le 2-hexylnonanol, le 2-heptyloctanol, l'alcool isotridécylique et/ou leurs mélanges.

10. Compositions selon au moins l'une quelconque des revendications 1 à 9, **caractérisées en ce que** les alcools non ramifiés b2) sont des alcools gras, de préférence l'alcool laurylique, myristylique, cétylique, stéarylique, eicosanylique et/ou béhénylique, et/ou le nonanol, l'undécanol, le tridécanol, le pentadécanol et/ou l'heptadecanol.

11. Compositions selon au moins l'une quelconque des revendications 1 à 10, **caractérisées en ce que** la teneur en polyols c), par rapport aux compositions finies, va de 0,5% à 20% en poids, de préférence de 0,5% à 10% en poids.

12. Compositions selon au moins l'une quelconque des revendications 1 à 11, **caractérisées en ce que** les polyols c) sont des glycols, de préférence l'éthylèneglycol et le propylèneglycol, des butanediols, de préférence le 1,3-butanediol, la glycérine, la thioglycérine, le sorbitol, le xylitol et le mannitol.

13. Compositions selon au moins l'une quelconque des revendications 1 à 12, **caractérisées en ce qu'**elles contiennent des monoalcools non ramifiés ou ramifiés contenant 1 à 4 atomes de carbone, de préférence l'éthanol, le propanol, l'isopropanol, le butanol; l'isobutanol et/ou le t-butanol, de manière plus particulièrement préférée l'isopropanol.

14. Compositions selon la revendication 13, **caractérisées en ce qu'**elles contiennent, par rapport aux compositions finies, moins de 5% en poids, de préférence moins de 1% en poids de monoalcools non ramifiés ou ramifiés contenant 1 à 4 atomes de carbone.

15. Compositions selon au moins l'une quelconque des revendications 1 à 14, **caractérisées en ce que** leur point d'écoulement se situe en dessous de 100°C, de préférence en dessous de 95°C, de manière plus particulièrement préférée en dessous de 90°C et de manière tout particulièrement préférée en dessous de 85°C.

16. Compositions selon au moins l'une quelconque des revendications 1 à 15, **caractérisées en ce que** leur point d'inflammation se situe au-dessus de 80°C, de préférence au-dessus de 100°C.

17. Procédé de préparation de compositions selon au moins l'une quelconque des revendications 1 à 16, **caractérisé en ce que** l'on prépare un mélange contenant
i) au moins un composé ammonium quaternaire a), comprenant éventuellement un monoalcool ramifié ou non ramifié contenant 1 à 4 atomes de carbone ;
ii) un mélange d'au moins un alcool ramifié b1) contenant 8 à 36 atomes de carbone et d'au moins un alcool non ramifié b2) contenant 8 à 36 atomes de carbone,
iii) au moins un polyol c) et
iv) éventuellement au moins un monoalcool non ramifié ou ramifié contenant 1 à 4 atomes de carbone.

18. Procédé de préparation d'une composition selon au moins l'une quelconque des revendications 1 à 16, **caractérisé en ce que** l'on alkyle
i) au moins une amine tertiaire NR₁R₂R₃, dans laquelle R₁ représente un groupe alkyle ou alcényle non ramifié ou ramifié contenant 12 à 36 atomes de carbone, un groupe R₅CONH(CH₂)ₙ- ou un groupe R₅COO (CH₂)ₙ-, dans lesquels R₅ représente un groupe alkyle ou alcényle contenant 12 à 36 atomes de carbone et n représente un nombre allant de 1 à 8 et R₂ et R₃ peuvent être, indépendamment l'un de l'autre, identiques ou différents et représentent CH₃-, CH₃CH₂-, CH₃CH₂CH₂-, CH₃CH₂CH₂CH₂- ou -CH₂CH₂(OH),
au moyen de
ii) au moins un agent alkylant, choisi parmi
a) R₄X, où R₄ représente CH₃-, CH₃CH₂-, CH₃CH₂CH₂-, CH₃CH₂CH₂CH₂- ou -CH₂CH(OH)CH₂(OH) et X représente Cl, I, Br, OSO₃H ou un méthosulfate et/ou
b) l'oxyde d'éthylène et un acide HX, dans lequel X représente Cl, I, Br, OSO₃H, un citrate ou un lactate,
en présence de
iii) un mélange d'au moins un alcool ramifié b1) contenant 8 à 36 atomes de carbone et d'au moins un alcool non ramifié b2) contenant 8 à 36 atomes de carbone et
iv) au moins un polyol c) et
v) éventuellement au moins un monoalcool non ramifié ou ramifié contenant 1 à 4 atomes de carbone.

19. Utilisation d'une composition selon au moins l'une quelconque des revendications 1 à 16 pour la fabrication de produits cosmétiques, dermatologiques et pharmaceutiques, de préférence des produits de soins capillaires.

20. Utilisation selon la revendication 19, **caractérisée en ce que** les produits de soins capillaires sont des shampooings, des après-shampooings à rincer, des crèmes de rinçage, des lotions de rinçage, des soins capillaires, des produits de teinture et de coloration capillaires, des produits de permanente, des gels capillaires et des produits coiffants sous forme d'aérosol, de pulvérisation et de liquide.
